# EUROPEAN PATENT APPLICATION

(11) **EP 0 867 114 A1**
(43) Date of publication of application: **30.09.1998**
(21) Application number: 96932037.3
(22) Date of filing: 27.09.1996
(51) Int. Cl.: A01K 67/027

(54) **METHOD FOR PREPARING TRANSGENIC ANIMAL**

(30) Priority: 29.09.1995 JP 275110/95
(71) Applicant: Hoechst Marion Roussel, Ltd., Tokyo 107-8465 (JP); Ogawa, Shyoso, Tokyo 156 (JP); Iwaya, Makoto, Tokyo 154 (JP)
(72) Inventor: OGAWA, Shyoso, Tokyo 156 (JP); IWAYA, Makoto, Tokyo 154 (JP); SATOH, Masahiro, Kanagawa 259-11 (JP); TADA, Norihiro, Saitama 336 (JP)
(74) Representative: Macchetta, Francesco
(86) International application number: JP9602828
(87) International publication number: WO9711597

(57) **Abstract**

A method for preparing a sperm containing an exogenous DNA, wherein the exogenous DNA together with liposome for the purpose of transduction of DNA into mammalian cells is injected repeating three times or more into a testis of a mature non-human vertebrate male and the sperm containing the exogenous DNA is produced in the testis of said male. It can be convenient and efficient to prepare a non-human transgenic vertebrate by mating a sperm containing an exogenous DNA with a female naturally, or mating an egg which has not yet been fertilized by artificial insemination or *in vitro* fertilization.

## Description

### Field of the Invention

The present invention relates to a convenient and efficient method for preparing transgenic animal. Specifically, the present invention relates to a method for preparing a sperm containing an exogenous DNA, wherein an exogenous DNA is injected into a testis of a mature non-human vertebrate male and then a sperm containing said exogenous DNA is produced in said testis. Moreover, the present invention relates to a method for preparing a transgenic non-human vertebrate animal, wherein said sperm containing the exogenous DNA thus obtained as described above is allowed to fertilize with an egg of non-human vertebrate animal as described above which has not been fertilized yet.

Today transgenic animals is being applied to many fields. For example, it has become extremely important as human disease model animals for the analysis of the tissue- and stage-specificities of gene expression, analysis of the physiological function of genes, and pathological analysis and medical effective tests. The most established method for preparing such animals is a microinjection method by using a micromanipulator to inject an exogenous DNA into a pronucleus of a fertilized egg (Palmiter and Brinster, *Annu. Rev. Genet*., 20:465-499, 1986). The microinjection method is performed routinely in some laboratories, but a certain problems are encountered when it is used to prepare a transgenic animal in general. For example, microinjection of an exogenous DNA is time-consuming and the operation requires specialized skills. Microinjection requires the use of an expensive micromanipulator. Moreover, in some species of animals, the nucleus of an egg to be injected of some species cannot be seen under a microscope, so it should be prepared by extra troublesome procedures involving centrifugation and other steps. Even after all this is done, the success rate in preparing a transgenic animal is subject to considerable variation.

On the other hand, some methods of preparing a transgenic animal without microinjection have been attempted. For example, there is a method for preparing transgenic animals in which a mouse sperm and an exogenous DNA are mixed together in a test tube and then said sperm is used to fertilize an unfertilized egg. Lavitrano *et al*. (Cell, 57:717-723, 1989) reported that they had used this method successfully, but up to the present, others have not been able to duplicate their results practically (Brinster *et al*., *Cell*, 59:239-241, 1989).

Another method attempted by Bachiller *et al*. is an improvement on the method of Lavitrano *et al*. described above, in which a mouse sperm is cultured together with a complex. comprising an exogenous DNA and liposome in order to improve the uptake of the exogenous DNA by an isolated sperm; the sperm is then used to fertilize unfertilized eggs, but no exogenous DNA was detected in the fertilized eggs (Mol. Reprod. Develop., 30:194-200, 1991). Namely, this method failed in preparing a transgenic animal. This method, although not having developed yet for a practical application, offers a certain advantage over the aforementioned microinjection method in that it does not require a special operation for microinjection nor does it require an expensive micromanipulator.

The present inventors have taken different approaches in their attempts to develop a so-called sperm-mediated gene transfer method, in which an exogenous DNA is transmitted to the egg through the agency of a sperm. For example, an exogenous DNA that has been precipitated by calcium phosphate is injected through a syringe directly into testes of a male animal (Sato *et al*., Animal Biotech., 5:19-31, 1994). The testes contain sperm cells in many different stages of differentiation, ranging from undifferentiated spermatogonia (which are the undifferentiated precursor for spermatozoa) through the spermatocytes, which are in the process of differentiation, to the testicular spermatozoa, which are even more differentiated. Testicular spermatozoa do not exhibit the vigorous motility characteristic of a sperm, but as they are transported through the epididymis they begin to exhibit this activity. Generally, an exogenous DNA that has been precipitated by calcium phosphate is readily taken up by cultured mammalian cells when the two are cultured together. It is known that phenotypical transformation may occur as a result of this process (Wigler *et al., Cell,* 11:223-232, 1977). It is thus conceivable that the injection of a calcium phosphate-precipitated exogenous DNA into a test is could induce phenotypical transformation of a testicular spermatozoa through the introduction of the exogenous DNA. Then, by releasing the phenotypically transformed sperm (containing the exogenous DNA) to fertilize eggs in the oviduct of a female animal, it is conceivable that the exogenous DNA will be transmitted to the chromosomes of the fertilized egg. Thus, a transgenic fertilized egg will be prepared. If this method succeeds in the preparation of transgenic animals, it will be possible to introduce an exogenous DNA into 100,000 sperm cells or more at once, providing a far more practical method than the difficult procedure of microinjection of an exogenous DNA into fertilized eggs such as a domestic animal. In addition, the use of a sperm containing an exogenous DNA permits other advantages, such as the ability to freeze and thaw the sperm for use at a more convenient time, and lends itself to the use of artificial insemination or *in vitro* fertilization and other techniques.

Based upon this hypothesis, the present inventors experimentally injected an exogenous DNA precipitated by calcium phosphate directly into testes of male mice. As a result, the presence of the exogenous DNA in the ejaculated sperm was confirmed, but no exogenous DNA was detected in the eggs after fertilization (Sato *et al*., Animal Biotech., 5:19-31, 1994).

### Detailed Description of the Invention

An object of the present invention is to provide a convenient and efficient method for preparing transgenic animals whereby a large number of transgenic animals may be prepared within a short period, but which does not require a conventional microinjection method. It is expected that, through this invention, it will be possible to solve the hitherto difficult problem of obtaining large transgenic domestic animals, such as cattle.

The present invention relates to a method for preparing an exogenous DNA together with liposome for injecting into a testis of a mature non-human vertebrate male and then producing a sperm containing said exogenous DNA in a testis of said male.

Moreover, the present invention relates to a method for preparing a transgenic non-human vertebrate animal, wherein an exogenous DNA is injected into a test is of a mature non-human vertebrate male after forming a complex with liposome for the purpose of transduction of DNA into mammalian cells, and a sperm is produced in a testis of said male, and then said male is allowed to mate with a female of which egg has not been fertilized yet.

The method of this invention is characterized by mixing an exogenous DNA together with liposome for the purpose of transduction of DNA into mammalian cells, and injecting it directly into a tesits of a mature non-human vertebrate male.

In this specification, "exogenous DNA" refers to "plasmid DNA." The type of an exogenous DNA that is introduced can be changed in order to prepare a desired transgenic animal. Liposome, which is formed to a complex by mixing with an exogenous DNA, can be any commerically available liposome sold for the purpose of transduction of DNA into mammalian cells (cationic liposome). For these liposome, liposome which contains LIPOFECTIN™ : N-[1-(2,3-dioleyloxy)propy]-N,N,N-trimethylammonium chloride (DOTMA) and dioleoyl phosphatidylethanolamine (DOPE) at a ratio of 1:1 (w/w) is preferably used. Liposome such as TransIT™-LT1 MIR2310, TransIT™-LT2 MIR2320, TransIT™-LT100 MIR2100 (Takara Shuzo), Genetransfer 074-03621, TRANSFECTAM 561-36591 (Wako Pure Chemical Industries, Ltd.) can be also used. Forming to a complex with an exogenous DNA and liposome is carried out according to a conventional method; for example, an exogenous DNA is added to a solution containing liposome, mixed and incubated.

A liposome-exogenous DNA complex is directly injected into a testis of mature non-human vertebrate male. The number of injection is carried out preferably three times or more. The mature males are anesthetized and the solution containing liposome described above is injected directly from the scrotum into both testes. The injection procedure is repeated at four day intervals for at least three times. On day 2 from the final injection of the liposome-exogenous DNA complex, a sperm containing the exogenous DNA is produced in the testis of said male.

A transgenic animal can be prepared by mating said males with females of whom estrus has been induced by gonadotropic hormone, making the females pregnant and delivered.

A transgenic animal can be prepared by artificial insemination or *in-vitro* fertilization using a sperm produced by the test is of a mature non-human vertebrate male that has been subjected to intratesticular injection of the exogenous DNA and by making the females pregnant and delivered.

The method of the present invention can be applied to efficiently produce non-human vertebrate animals such as experimental animals; a mouse, a rat, a rabbit, a dog, and a cat, etc. as well as domestic animals; a horse, a cattle, a pig, a goast and a sheep, etc.

A transgenic animal obtained by the method of the present invention can be raised and bred under normal conditions of animal husbandry and using ordinary animal feed.

Whether a fertilized egg or an animal obtained contains the exogenous DNA or not is confirmed by the following experiment as illustrated.

Some of the females are sacrificed on the day when copulation is confirmed (determined as the first day of pregnancy), and the intrauterine sperm and the fertilized eggs (single-cell embryos) in the oviduct are sampled. Said samples are analyzed for the presence of an exogenous DNA. The other females are allowed to live until midjestational stages through pregnancy or allowed to come to term. For those allowed to live until midjestational stages through pregnancy, the fetus is extirpated, the DNA extracted, and the rate of incorporation of an exogenous DNA in the fetal chromosome is determined by Southern blotting (Southern, *J Mol Biol*., 98:503-517, 1975) or PCR method (Saiki *et al., Science*, 239:487-491, 1988). In newborn individuals, the presence of an exogenous DNA is determined from DNA extracted from the tail on the fifth day after birth.

Mature transgenic individuals (referred to as a founder or F0 generation) are allowed to mate with normal animals to produce offspring. DNA from the second generation (F1) individuals is analyzed by Southern blotting or PCR method to determine whether an exogenous DNA has been transmitted from F0 to F3.

### BRIEF DESCRIPTION OF THE DRAWINGS

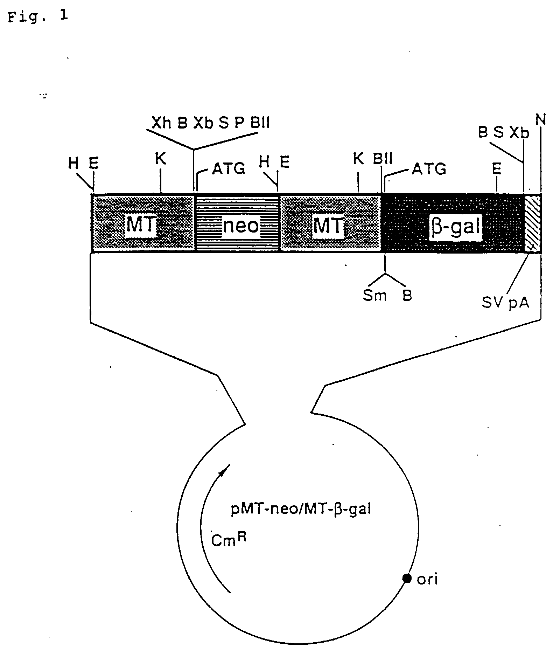

Fig. 1 shows a plasmid map for the plasmid pMT-neo/MT-beta-gal, used for DNA transduction.

This has the DNA sequence coding for mouse metallothionein-I promoter (MT), the gene for neomycin resistance (neo), and the DNA sequence coding for the beta-galactosidase gene (beta-gal) downstream of the repeating DNA sequence for the mouse metallothionein-I promoter.

Explanation of symbols used in the diagram: H, *Hind*III restriction endonuclease site; E, *Eco*RI restriction endonuclease site; K, *Kpn*I restriction endonuclease site; Xh, *Xho*I restriction endonuclease site; B, *Bam*HI restriction endonuclease site; Xb, *Xba*I restriction endonuclease site; S, *Sal*I restriction endonuclease site; P, *Pst*I restriction endonuclease site; B II, *Bgl*II restriction endonuclease site; Sm, *Sma*I restriction endonuclease site; ATG, protein translation initiation site; SV pA, SV40 poly(A)addition signal site; N, *Not*I restriction endonuclease site; Cm^{R}, gene for chloramphenicol resistance; ori, replication origin.

Fig. 2 is a schematic drawing of the operation for direct injection of the liposome-exogenous DNA complex into both testes of adult male ICR mice.

Fig. 3 shows a photograph of PCR analysis (using MI499 beta-gal and MI500 beta-gal as primers) of genomic DNA derived from an intrauterine sperm deposited by a male that had received a series of three injections of liposome-exogenous DNA complex. The symbol "m" indicates a molecular weight marker.

Figs. 4 (a) and 4 (b) present microphotographs of blastocysts fertilized by males that had received a series of three injections of liposome-exogenous DNA complex. Fig. 4 (c) is a photograph of the embryos on the thirteenth day of pregnancy. The arrow in Fig. 4 (a) points to blastocysts that do not exhibit any beta-gal enzymatic activity. The symbol "Tg" in Fig. 4 (c) indicates an embryo that has exogenous DNA and exhibits beta-gal enzymatic activity, whereas the "Non-Tg" indicates a non-transgenic embryo that does not show any beta-gal enzymatic activity.

Fig. 5 is a photograph of PCR analysis (using MI499 beta-gal and MI500 beta-gal as primers) of genomic DNA derived from blastocysts fertilized by males that had received a series of three injections of liposome-exogenous DNA complex. The symbol "m" indicates a molecular weight marker.

Fig. 6 is a photograph of PCR analysis (using MI499 beta-gal and MI500 beta-gal as primers) of genomic DNA derived from the tail of embryos on the thirteenth day of pregnancy fertilized by males that had received three injections of liposome-exogenous DNA complex. The symbol "M" indicates a molecular weight marker.

Fig. 7 shows the pedigree of descendants of Male No. 7 whose testes were injected with an exogenous DNA.

### EXAMPLE

The following examples illustrate the method of the invention more concretely. However, it should not be construed that the invention is limited to these specific examples.

### EXAMPLE 1 Preparation of plasmid pMT-neo/MT-beta-gal for DNA transduction

Mouse metallothionein-I promoter (MT) fragment (approximately 1.9 kb) was digested by *Eco*RI/*Bgl*II from pMGH (Palmiter *et al*.*, Nature*, 300:611-615, 1982), cleaved, and isolated. This was then introduced onto the *Eco*RI/*Bgl*II restriction enzyme site of plasmid pHSG396 (approx. 2.3 kb; Takeshita *et al., Gene*, 71:9-18, 1988). This is referred to as pMT/1. Then a gene for neomycin resistance (approximately 1.5 kb; "neo") was isolated from plasmid pHSG294 (Brady *et al*., *Gene*, 27:223-232, 1984) by digestion with *Bgl*II/HindIII restriction endonuclease and introduced onto the *Bgl*II/*Hin*dIII site of pMT/1 to form the vector pMT/neo-1. This vector contains the neo-expressing unit (MT-neo) where neo expression is under the control of MT. An expression unit is an assemble for expressing a gene, and normally comprises a promoter and a gene (cDNA or genomic DNA).

Separately, a beta-gal expressing unit (MT-beta-gal) of approximately 5 kb, being a fusion of MT and an *Escherichia coli*-derived beta-gal gene (approx. 3 kb, including an SV40 poly(A)addition site on the 3'-terminal), was isolated from pMT/beta-gal-1 (Sato *et al., Mol. Reprod. Develop*., 34:349-356, 1993), and this was inserted onto the *Hin*dIII restriction enzyme site on pMT/neo-1 (downstream MT-neo). At this time, a *Not*I linker (Boehringer Mannheim GmbH) was put on the 3'-terminal of the MT-beta-gal. The plasmid obtained by this operation was named "pMT-neo/MT-beta-gal" (approx. 10 kb; refer to Fig. 1). This plasmid contained beta-gal expressing units and neo-expressing units in a serially linked form of MT-neo and MT-beta-gal. In this example the plasmid for DNA transduction was formed by pMT-neo/MT-beta-gal, but it would also be possible to use a unit expressing a different gene of interest in place of MT-neo or MT-beta-gal.

### EXAMPLE 2 Injection of the liposome-exogenous DNA (pMT-neo/MT-beta-gal) complex into the testis of male mice and extraction of an exogenous DNA from an intrauterine sperm and blastocysts obtained after coitus of said mice

The intratesticular sperms were subjected to phenotypical transformation by the injection of the liposome-exogenous DNA complex into the testis. After the sperms had been ejaculated during coitus, the intrauterine sperms and the eggs fertilized by the sperms were examined for the presence of exogenous DNA.

First, *Not*I restriction endonuclease was used to cleave plasmid pMT-neo/MT-beta-gal DNA, giving a linearized DNA. Approximately 5 µg of the linearized DNA was mixed at a 1:1 ratio with Lipofectin™ (GIBCO-BRL, dissolved in PBS buffer, pH 7.2) at room temperature, and this was allowed to stand for 15 minutes. The manufacturer's instructions were followed for this procedure. In this process the exogenous DNA (pMT-neo/MT-beta-gal) becomes surrounded by a lipid layer of liposome and forms a so-called liposome-exogenous DNA complex. A control solution was prepared by mixing liposome at a 1:1 ratio with PBS.

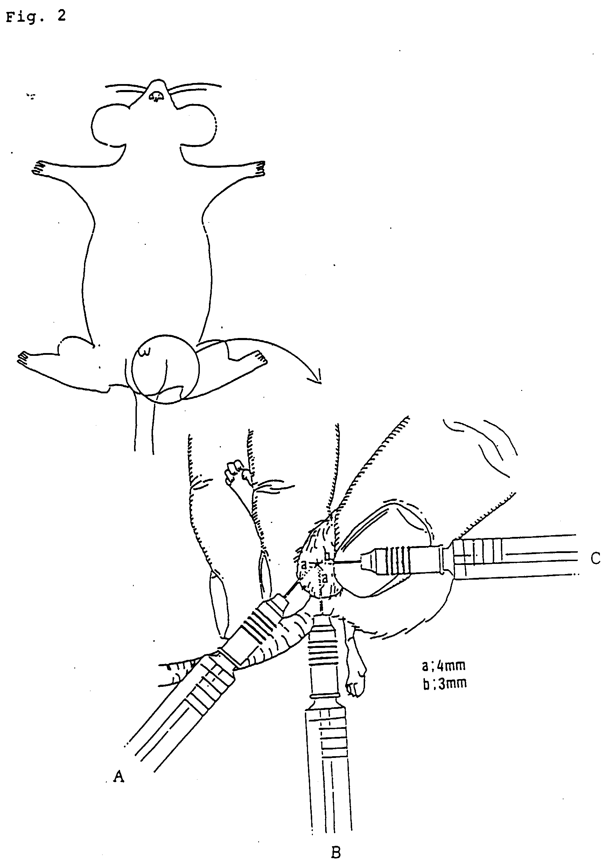

The liposome-exogenous DNA complex and the control solution were each drawn up using a syringe with 1/6 gauge needle and approximately 20 µl were injected directly through the scrotum into both testes of anesthetized adult ICR male mice (age: 8-10 weeks, obtained from CLEA Japan, Inc.). A schematic drawing of the injection procedure is shown in Fig. 2. After injection of the liposome-exogenous DNA complex, the males were allowed to mate with ICR females (age: 8-10 weeks, obtained from CLEA Japan, Inc.; estrus was induced by gonadotropic hormones) at the second day after DNA injection. Then fertilized eggs (1-cell-embryos) and the intrauterine sperms were recovered from the oviduct and the uterus, respectively. The cumulus oophorus cells were removed from the recovered fertilized eggs by hyaluronidase and were then cultured for three days at 37°C in an atmosphere of 5% CO₂/95% air, on 100 µl of M16 culture fluid (Whittingham, *J. Reprod. Fert*., 14(Supple):7-21, 1971) containing 1 µM CdCl₂ (for the purpose of increasing MT activity), until blastocysts had formed. Then DNA analysis by PCR method was used to determine the presence of an exogenous DNA on the cultured blastocysts. Also, some of the blastocysts were analyzed histochemically for beta-gal activity to determine if the introduced gene was being expressed. The method of Saiki *et al*. (*science,* 239:487-491, 1988) was used for DNA analysis by PCR method. At this time, two primer sets were used: the first comprised the MI499 beta-gal (SEQ ID NO.: 1) and the MI500 beta-gal (SEQ ID NO.: 2), both of which recognize the region on the 3' end of the beta-gal gene (*Molecular Cloning---A Laboratory Manual*, CSH, NY, 1982); the second comprised the MI1511 neo (SEQ ID NO.: 3) and the MI1512 neo (SEQ ID NO.: 4), both of which recognize the region on the 5' end of the neo gene (*Gene,* 19:327-336, 1982). PCR reaction conditions were as follows: 1 µl (approximately 1 µg) of genomic DNA or solution containing blastocysts was added to 9 µl of reaction mixture (10 mM Tris-Cl, pH 8.3, 1.5 mM MgCl₂, 50 mM KCl, 0.01% (w/v) gelatin, 200 µM dATP, dTTP, dGTP and dCTP, 50 pM of each of primers, and 0.1 µl of Taq polymerase), and this was reacted for 36 PCR cycles. After denaturing for one minute at 95°C, the reaction mixture was annealed at 58°C for one minute, followed by an extension reaction at 75°C for four minutes. The reaction mixture was then subjected to electrophoresis on 4% agarose gel, and the gel was stained with ethidium bromide. After staining, the presence of the amplified exogenous DNA was determined under an ultraviolet illumination.

Histochemical analysis of beta-gal was conducted as follows: specimens (blastocysts) were fixed at room temperature for five minutes in an intimate mixture of 1.25% glutaraldehyde in PBS, then washed three times in droplets of 50 µl PBS containing 0.05% bovine serum albumin (BSA). Subsequently the specimens were reacted for approximately 24 hours at 37°C in PBS (50 µl) containing 1.2 mM 5-bromo-4-chloro-3-indolyl-beta-D-galactopyranoside (X-Gal), 1 mM MgCl₂, 0.1% Triton X-100, 3 mM K₄Fe[CN]₆·3H₂O (P3289; Sigma), and 3 mM K₃Fe[CN]₆ (P3667, Sigma). This operation causes blue cytoplasmic staining of specimens that exhibit beta-gal activity. No staining occurs in cytoplasm of specimens that exhibit no beta-gal activity.

The recovered intrauterine sperms were subjected to a procedure to isolate genomic DNA. The method of Sato *et al.* (*Animal Biotech*, 5:19-31, 1994) was used to isolate the genomic DNA. Specifically, the intrauterine sperms deposited by light centrifugation was placed in 700 µl lysis buffer (150 µg/ml proteinase K (No. 24568, Merck), 0.5 mg/ml Pronase E (Kaken Kagaku Company), 100 mM NaCl, 0.4% SDS, 10 mM Tris buffer, 10 mM EDTA, pH 8.0), and allowed to belysed at 37°C for 24-48 hours. Then 100 µl phenol was added to the lysis mixture and the supernatant was extracted. The supernatant was then treated at 37°C for 30 minutes in 80 µg/ml ribonuclease A (RNase A), thus decomposing the RNA present in the mixture. This supernatant was then treated with 700 µl of isopropanol, yielding the DNA as precipitate. The DNA precipitate was wiped up using a Gilson Yellow tip, washed in 70% ethanol, and dissolved in 70 µl of TE buffer (10 mM Tris buffer and 1 mM EDTA, pH 7). The genomic DNA derived from intrauterine sperms was subjected to PCR analysis using two different primer sets to determine the presence of exogenous DNA.

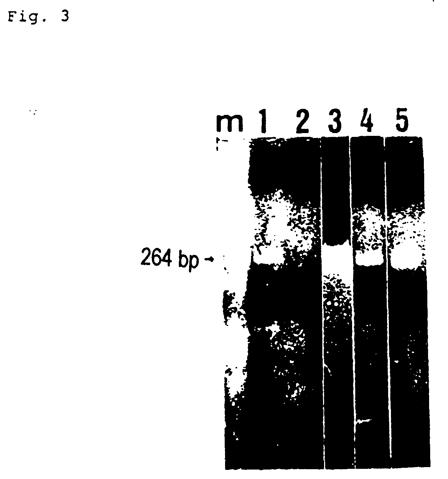

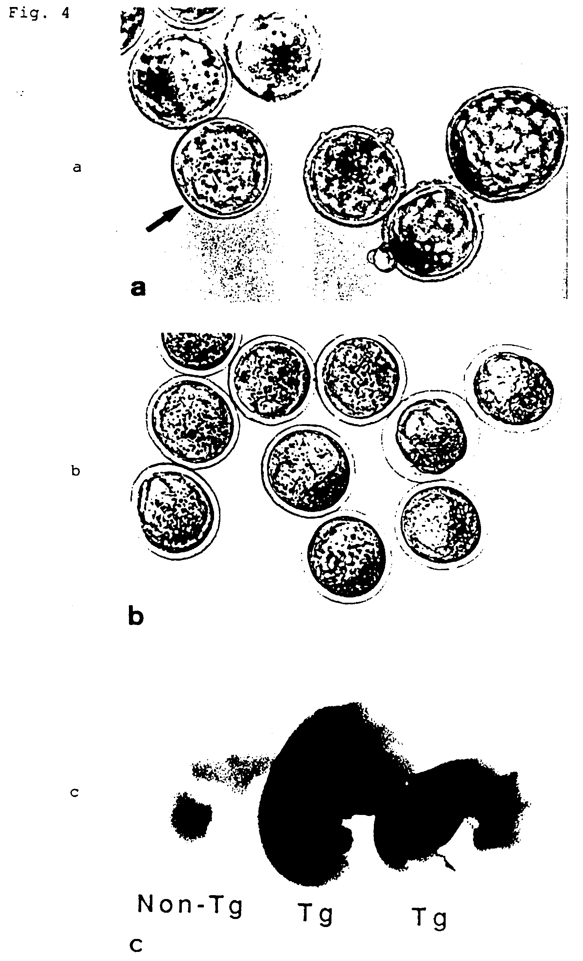

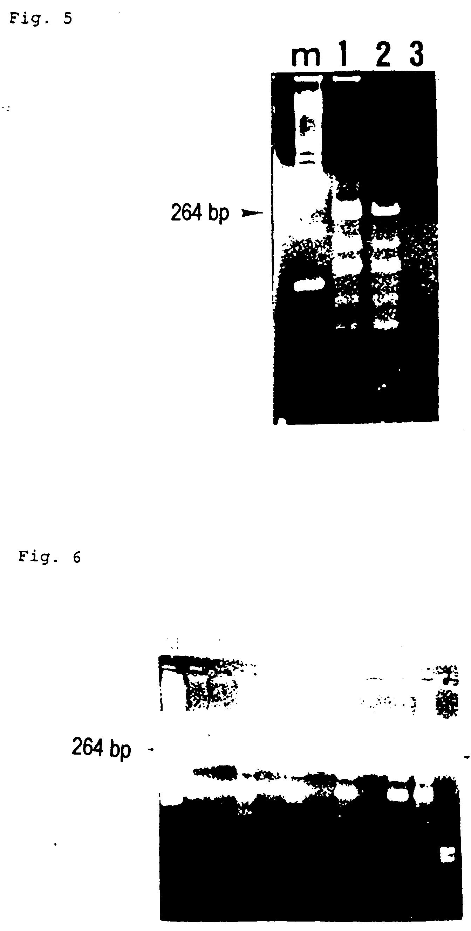

Control solution or solution containing a liposome-exogenous DNA (pMT-neo/MT-beta-gal) was injected into the test is of male animals and these mice were then mated with estrus females in the second day after DNA injection, in order to isolate fertilized eggs. Analysis of beta-gal expression was then performed using blastocysts derived from these fertilized eggs (first injection). Four days after the first injection of the liposome-exogenous DNA, the test is was injected a second time with control solution or with the liposome-exogenous DNA. Two days after the second injection of the liposome-exogenous DNA, the males were mated with estrus females. The fertilized eggs were then isolated and the genomic DNA was subjected to DNA analysis and expression analysis, as was done for the group which had received only one injection. Furthermore, four days after the second testicular injection of DNA, another injection of liposome-exogenous or control solution was performed (third injection). Generally the first injection was made from the direction labeled "A" in Fig. 2, with the syringe inserted from the surface of the scrotum to a depth of approximately 4 mm before the complex was injected. The second injection was made from direction labeled "B" in Fig. 2, with the syringe inserted from the surface of the scrotum to a depth of approximately 4 mm before the complex was injected. The third injection was made from direction labeled "C" in Fig. 2, with the syringe inserted from the surface of the scrotum to a depth of approximately 3 mm before the complex was injected. On the second day after the third injection of the liposome-exogenous DNA, the males were mated with estrus females. The fertilized eggs and the intrauterine sperms were then isolated, the presence exogenous DNA was determined by PCR method, and the expression of an exogenous DNA was histochemically determined for beta-gal activity. The results of these tests are shown in Table 1. Fig. 3 shows the results of testing for the presence of an exogenous DNA in the intrauterine sperm after a full series of three injections. In Fig. 3, the first lane shows the results of PCR analysis of a genomic DNA obtained from testis on the fourth day after the third injection of the liposome-exogenous DNA complex; the band observed in this lane corresponds to that expected from the 264 bp segment derived from the exogenous DNA. The second lane shows the results of PCR analysis of a genomic DNA derived from a normal mouse; no bands are observed that could be attributed to a 264 bp segment derived from the exogenous DNA. The third lane shows the results of PCR analysis of 10 pg purified exogenous DNA (pMT-neo/MT-beta-gal); the band observed in this lane corresponds to that expected from the 264 bp segment derived from the exogenous DNA. The fourth and fifth lanes show the results of PCR analysis of the genomic DNA obtained from the intrauterine sperms released by a male on the second day after having received the third injection of a DNA-liposome complex; the bands corresponds to those expected for a 264 bp segment derived from the exogenous DNA. The samples for lanes four and five were obtained from different male mice. Figs. 4 (a) and 4 (b) show the results of histochemical analysis of beta-gal activity in blastocysts fertilized by males that had received a series of three injections. The blastocysts shown in Fig. 4 (a) were obtained by natural mating two days after the male had received the third of a series of DNA-liposome complex injections; these blastocysts were reacted in the presence of X-Gal, with the result that most were stained. The arrows point to unstained blastocysts. Fig. 4 (b) shows blastocysts that were obtained by natural mating on the second day after the male had received an injection of control solution; these blastocysts were reacted in the presence of X-Gal, but none of them were stained. Fig. 4 (c) is a photograph of the embryos on the thirteenth day of pregnancy obtained after natural mating on the second day after the male had received the third of a series of DNA-liposome complex injections; after the embryos were reacted in the presence of X-Gal, two were stained (indicated by the symbol "Tg" for "transgenic") and one was not stained (indicated by "non-Tg" for "non-transgenic"). Fig. 5 shows a photograph of PCR analysis (using MI499 beta-gal and MI500 beta-gal as the primers) of the genomic DNA derived from blastocysts fertilized by males that had received a series of three injections of the liposome-exogenous DNA complex. Lanes one and two show the results of analyzing genomic DNA derived from 10 and 5 blastocysts, respectively, that had been obtained by mating on the second day after the male had received the third of a series of DNA-liposome complex injections; a band is observed which corresponds to that predicted for the 264 bp segment derived from exogenous DNA. Lane three shows the results of PCR analysis of the genomic DNA from 10 blastocysts obtained from a normal mouse; in this case no bands are observed that would result from a 264 bp segment of the exogenous DNA. The results are summarized below.
(1) The presence of the exogenous DNA was observed in the intrauterine sperms obtained after natural mating of a female with a male who had received intratesticular injection of liposome-exogenous DNA complex.
(2) The presence of the exogenous DNA was observed in blastocysts derived from fertilized ova obtained after natural mating of a female with a male who had received intratesticular injection of liposome-exogenous DNA complex. This suggests that the exogenous DNA introduced into the test is was transmitted *via* the sperms to the ova.
(3) The presence of the exogenous DNA in the blastocysts was demonstrated by histochemical analysis for beta-gal activity. Specifically, as the number of intratesticular injections of liposome-exogenous DNA complex increased, the proportion of blastocysts expressing beta-gal activity also increased.

### EXAMPLE 3 The detection of the exogenous DNA in the embryo (F0) obtained from matings that occur two days after intratesticular injection of liposome-exogenous DNA complex

This experiment was tested whether the exogenous DNA would still be present in embryos (on the thirteenth day of pregnancy) derived from eggs fertilized by sperms that had been subjected to phenotypical transformation by injection of liposome-exogenous DNA complex into the testis.

Male ICR mice that had received a series of three injections of control solution or liposome-exogenous DNA complex were mated with estrus female ICR mice (8-10 weeks old; estrus was induced by gonadotropic hormones). On the thirteenth day of pregnancy, corresponding to a midgestational stage, the embryos were extracted from the womb and genomic DNA was sampled from the tails. The genomic DNA was analyzed by PCR method described in Example 2. The results are shown in Table 2 and Fig. 6. In Fig. 6, no formation of a band predicted by the 264 bp size of the exogenous DNA was observed in lanes 1, 2, 4, 6, and 8, which are accordingly interpreted as non-transgenic. The formation of a band predicted by the 264 bp size of the exogenous DNA was observed in lanes 3, 5, 7, 9, and 10, which are accordingly interpreted as transgenic. The formation of a band predicted by the 264 bp size of the exogenous DNA was not observed in lane C, which shows the results of PCR analysis of genomic DNA from the tail of a normal mouse.
The results are summarized below.
(1) The presence of the exogenous DNA was determined in PCR analysis of genomic DNA in 4 out of 14 embryos (28.6%) that were examined in the experimental group. These results are similar to the results of PCR analysis using primer sets that recognize the neo gene.
(2) Out of 53 embryos examined in the experimental group, 18 (34.0%) were determined to show beta-gal activity.
(3) No exogenous DNA was present in any out of 10 embryos examined in the control group. Nor did any out of 21 embryos in the control group examined for beta-gal activity exhibit such activity.

These results demonstrate that the invented method successfully yielded transgenic embryos that had conserved the exogenous DNA.

### EXAMPLE 4 Transmission of the exogenous DNA to the successor generation (F1) from the transgenic individual (F0) sired by a male subjected as an adult to intratesticular injection of liposome-exogenous DNA complex

Since it had been confirmed that the exogenous DNA was present in individuals grown from ova fertilized by sperms that had been subject to phenotypical transformation through intratesticular injection of liposome-exogenous DNA complex, further tests were conducted to determine whether said exogenous DNA would be transmitted to succeeding generations or not.

First, liposome-exogenous DNA complex or control solution was injected three times into ICR male mice, who were then mated with estrus ICR females (8-10 weeks old, estrus was induced by gonadotropins) two days after the final injection. Samples were taken from the tails of offspring of this mating (F0) on the fourth week after birth, and subjected to DNA analysis by PCR method and Southern blotting. The method used to isolate the genomic DNA from the tail and the method of PCR analysis were identical to the same methods described in Example 2. The Southern blotting basically followed the method of Sato *et al.* (*Mol. Reprod. Develop*. 34:349-356, 1993). Specifically, 10 µg of genomic DNA was digested by *Eco*RI and *Bam*HI restriction endonucleases, and then placed on a 0.8% agarose gel. The DNA on the gel was transferred to a nylon membrane filter (GeneScreenPlus™; NEN Company, USA) under alkaline conditions. The nylon membrane filter was then subjected to Southern hybridization using a ³²P-labeled probe (DNA fragment from part of the neo gene or beta-gal gene). After Southern hybridization the filter was washed once in 0.1 x SSC/0.01% SDS for 30 minutes at 56°C and exposed tog Kodak XAR-5 film.

The results are shown in Table 2. In the experimental group, the presence of the exogenous DNA was determined in 25 out of 47 F0 individuals (53.2%). This indicates that the exogenous DNA that had been injected into the testis was transmitted *via* the sperms to the offspring.

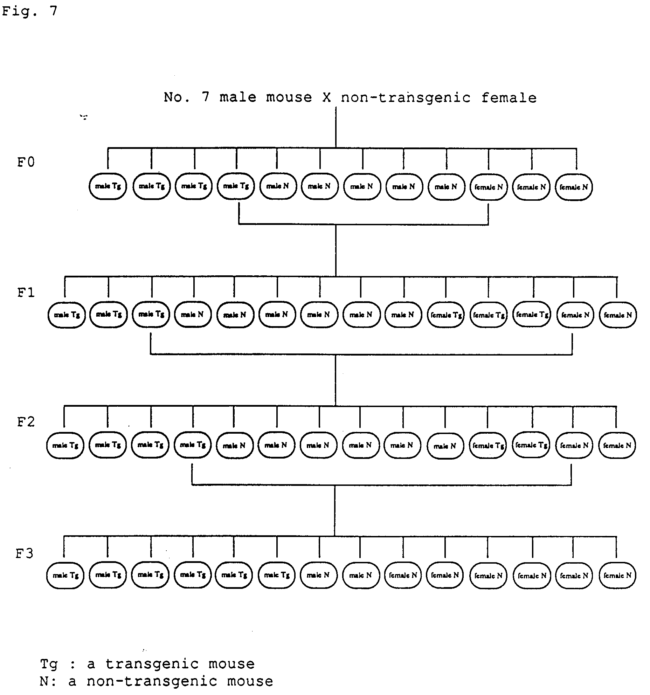

The second generation of offspring (F1) produced by the F0 transgenic animals was also examined for the presence of the exogenous DNA. Genomic DNA was extracted from the tails of F1 mice according to the procedure described in Example 2. The genomic DNA was subjected to PCR analysis and Southern blotting according to the methods described in Example 2 and Example 4. The partial results are shown in Fig. 7. In Fig. 7, the individuals having the exogenous DNA have been traced using PCR method on genomic DNA extracted from the tail on four-week-old individuals in F0 and F1 generations. Analysis of DNA in F0 descendants demonstrated that approximately 40% of F0 descendants were transgenic. Analysis of DNA from F1 descendants demonstrated that approximately 38% of F1 descendants were transgenic. This proportion indicates that the genomic DNA was probably transmitted to descendants in Mendelian fashion, which is exactly identical to the inheritance of the exogenous gene in the transgenic individuals obtained by the prior art method (microinjection).

**Table 1**

| Gene expression of exogenous DNA in blastocysts sired by males subjected to intratesticular injection of exogenous DNA | | | | |
|---|---|---|---|---|
| Number of injections of exogenous DNA | (x1) | (x2) | (x3) | Control (x3) |
| Number of 1-cell embryos recovered after mating, following intratesticular injection of exogenous DNA | 82 | 81 | 21 | 40 |
| Number of embryos developed to the two-cell stage (%) | 82 (100) | 81 (100) | 20 (95.2) | 40 (100) |
| Development to blastocyst (%) | 53 (64.6) | 61 (75.3) | 20 (95.2) | 38 (95.0) |
| Number of blastocysts exhibiting beta-gal activity / Number of all blastocysts tested | 1/53 | 25/61 | 16/20 | 0/38 |
| Ratio (%) of blastocysts exhibiting beta-gal activity | 1.9 | 41.0 | 80.0 | 0.0 |

**Table 2**

| Detection of exogenous DNA and its expression in embryos and newborns obtained from matings with females by males subjected to intratesticular injection of exogenous DNA | | |
|---|---|---|
| Number of injections of exogenous DNA | (x3) | Control (x3) |
| Number of 13-day-old embryos showing beta-gal activity / Number of all 13-day-old embryos obtained in the experiment | 18/53 | 0/21 |
| Ratio (%) of embryos showing beta-gal activity | 34.0 | 0 |
| Number of 13-day-old embryos with exogenous DNA / Number of all 13-day-old embryos obtained in the experiment | 4/14 | 0/10 |
| Ratio (%) of embryos with exogenous DNA | 28.6 | 0 |
| Number of newborns having exogenous DNA / Number of all newborns obtained in the experiment | 25/47 | 0/15 |
| Ratio (%) of newborns having exogenous DNA | 53.2 | 0 |

### EXAMPLE 5 Transmission of the exogenous DNA to the successor generation (F3) from the transgenic individual (F0)

Whether a liposome-exogenous DNA complex can be detected in the successor generations, F1, F2 and F3, or not was investigated by increasing the number of animals according to the same procedure as Example 4. The result is shown in Table 3 and Fig. 7.

**Table 3**

| | | | | |
|---|---|---|---|---|
| Number of injections or exogenous DNA | F0 | F1 | F2 | F3 |
| Number of newborns having exogenous DNA/ Number of all newborns obtained in the experiment | 39/107 | 28/74 | 14/45 | 14/52 |
| Ratio (%) of newborns having exogenous DNA | 36.4 | 37.8 | 31.1 | 26.9 |

Judging from the results, the invented method permits the preparation of a transgenic animal more conveniently and efficiently as shown in the similar result of Example 4.

### Industrially Applicable

The present invention relates to a method for preparing a non-human vertebrate transgenic animal, by injecting an exogenous DNA which is complexed with liposome, for the purpose of transduction of DNA into mammalian cells, into a testis of a mature non-human vertebrate male. The number of injection is carried out preferably three times or more. Then a transgenic animal can be prepared by mating said male with a female of which egg has not been fertilized yet by artificial insemination or *in-vitro* fertilization using a sperm produced by the test is of said male.

The method of preparing a transgenic animal is extremely important as a technology for preparing a domestic animal with effective gene and a human disease model animal for medical development. The established method for preparing such animals is a microinjection method by using a micromanipulator to inject an exogenous DNA into a pronucleus of a fertilized egg. However, a certain problems are encountered; this method is time-consuming, the number of treatment at once is limited, the operation requires specialized skills and it requires the use of an expensive micromanipulator.

By using the present method, an exogenous DNA can be injected into 100,000 or more sperm cells at once, which is extremely effective for a domestic animal of which egg is hard to handle by microinjection. Moreover, the present invention is industrially applicable to prepare a trangenic animal simply because a lot of sperm cells containing an exogenous DNA are subject to freeze and thaw for use at a more convenient time, and lend themselves to the use of artificial insemination or *in vitro* fertilization.

SEQUENCE LISTING

SEQ ID NO: 1:

SEQUENCE LENGTH: 21

SEQUENCE TYPE: nucleic acid

STRANDEDNESS: single

TOPOLOGY: linear

MOLECULE TYPE: other nucleic acid

ORIGINAL SOURCE: none

ORGANISM: none

STRAIN: none

FEATURE: primer for recognizing the nucleotides from numbers

4035 to 4055 on the beta-gal gene; called "MI499 beta-gal"

SEQUENCE DESCRIPTION: SEQ ID NO: 1:

GACCGCTGGG ATCTGCCATT G

SEQ ID NO: 2:

SEQUENCE LENGTH: 21

SEQUENCE TYPE: nucleic acid

STRANDEDNESS: single

TOPOLOGY: linear

MOLECULE TYPE: other nucleic acid

ORIGINAL SOURCE: none

ORGANISM: none

STRAIN: none

FEATURE: primer for recognizing the nucleotides from numbers

4278 to 4298 on the beta-gal gene; called "MI500 beta-gal"

SEQUENCE DESCRIPTION: SEQ ID NO: 2:

TACTGACGGG CTCCAGGAGT C

SEQ ID NO: 3:

SEQUENCE LENGTH: 26

SEQUENCE TYPE: nucleic acid

STRANDEDNESS: single

TOPOLOGY: linear

MOLECULE TYPE: other nucleic acid

ORIGINAL SOURCE: none

ORGANISM: none

STRAIN: none

FEATURE: primer for recognizing the nucleotides from numbers

1752 to 1767 on the neo gene; called "MI1511 neo"

SEQUENCE DESCRIPTION: SEQ ID NO: 3:

TCGTGGCTGG CCACGACGGG CGTTCC

SEQ ID NO: 4:

SEQUENCE LENGTH: 16

SEQUENCE TYPE: nucleic acid

STRANDEDNESS: single

TOPOLOGY: linear

MOLECULE TYPE: other nucleic acid

ORIGINAL SOURCE: none

ORGANISM: none

STRAIN: none

FEATURE: primer for recognizing the nucleotides from numbers

1846 to 1861 on the neo gene; called "MI1512 neo"

SEQUENCE DESCRIPTION: SEQ ID NO: 4:

GACAGGAGAT CCTGCC

SEQUENCE LISTING

(1) GENERAL INFORMATION:

(i) APPLICANT:
(A) NAME: Hoechst Marion Roussel Ltd.
(B) STREET: 17-51 Akasaka 2-Chome
(C) CITY: Minato-ky, Tokyo
(E) COUNTRY: Japan
(F) POSTAL CODE (ZIP): 107
(G) TELEPHONE: 0081 3 55716402
(H) TELEFAX: 0081 3 55716213

(A) NAME: OGAWA Shyoso
(B) STREET: 16-12 Akazutsumi 4-Chome
(C) CITY: Setagaya-ku, Tokyo
(E) COUNTRY: Japan
(F) POSTAL CODE (ZIP): 156

(A) NAME: IWAYA Makto
(B) STREET: 8-10 Mishuku 1-Chome
(C) CITY: Setagaya-ku, Tokyo
(E) COUNTRY: Japan
(F) POSTAL CODE (ZIP): 154

(ii) TITLE OF INVENTION: Method for preparing transgenic animal

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 96932037.3

(2) INFORMATION FOR SEQ ID NO: 1:

(i) SEQUENCE CHARACTERISTICS:
(A) LENGTH: 21 base pairs
(B) TYPE: nucleic acid
(C) STRANDEDNESS: single
(D) TOPOLOGY: linear

(ix) FEATURE:
(D) OTHER INFORMATION:/note= "primer for recognizing the
nucleotides from numbers 4035 to 4055 on the beta-gal gene;
called "M1499 beta-gal""

(xi) SEQUENCE DESCRIPTION: SEQ ID NO: 1:

GACCGCTGGG ATCTGCCATT G 21

(2) INFORMATION FOR SEQ ID NO: 2:

(i) SEQUENCE CHARACTERISTICS:
(A) LENGTH: 21 base pairs

(B) TYPE: nucleic acid
(C) STRANDEDNESS: single
(D) TOPOLOGY: linear

(ii) MOLECULE TYPE: other nucleic acid

(vi) ORIGINAL SOURCE:
(A) ORGANISM: none
(B) STRAIN: none

(ix) FEATURE:
(D) OTHER INFORMATION:/note= "primer for recognizing the
nucleotides from numbers 4278 to 4298 on the beta-gal gene;
called "M1500 beta-gal""

(xi) SEQUENCE DESCRIPTION: SEQ ID NO: 2:

TACTGACGGG CTCCAGGAGT C 21

(2) INFORMATION FOR SEQ ID NO: 3:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: other nucleic acid

(vi) ORIGINAL SOURCE:
(A) ORGANISM: none
(B) STRAIN: none

(ix) FEATURE:
(D) OTHER INFORMATION:/note= "primer for recognizing the
nucleotides from numbers 1752 to 1767 on the neo gene; called
"M1511 neo""

(xi) SEQUENCE DESCRIPTION: SEQ ID NO: 3:

TCGTGGCTGG CCACGACGGG CGTTCC 26

(2) INFORMATION FOR SEQ ID NO: 4:

(i) SEQUENCE CHARACTERISTICS:
(A) LENGTH: 16 base pairs
(B) TYPE: nucleic acid
(C) STRANDEDNESS: single
(D) TOPOLOGY: linear

(ix) FEATURE:
(D) OTHER INFORMATION:/note= "primer for recognizing the
nucleotides from numbers 1846 to 1861 on the neo gene; called
"M1512 neo""

(xi) SEQUENCE DESCRIPTION: SEQ ID NO: 4:

GACAGGAGAT CCTGCC 16

SEQUENCE LISTING

SEQ ID NO: 1:

SEQUENCE LENGTH: 21

SEQUENCE TYPE: nucleic acid

STRANDEDNESS: single

TOPOLOGY: linear

MOLECULE TYPE: other nucleic acid

ORIGINAL SOURCE: none

ORGANISM: none

STRAIN: none

FEATURE: primer for recognizing the nucleotides from numbers

4035 to 4055 on the beta-gal gene; called "MI499 beta-gal"

SEQUENCE DESCRIPTION: SEQ ID NO: 1:

GACCGCTGGG ATCTGCCATT G

SEQ ID NO: 2:

SEQUENCE LENGTH: 21

SEQUENCE TYPE: nucleic acid

STRANDEDNESS: single

TOPOLOGY: linear

MOLECULE TYPE: other nucleic acid

ORIGINAL SOURCE: none

ORGANISM: none

STRAIN: none

FEATURE: primer for recognizing the nucleotides from numbers

4278 to 4298 on the beta-gal gene; called "MI500 beta-gal"

SEQUENCE DESCRIPTION: SEQ ID NO: 2:

TACTGACGGG CTCCAGGAGT C

SEQ ID NO: 3:
SEQUENCE LENGTH: 26
SEQUENCE TYPE: nucleic acid
STRANDEDNESS: single
TOPOLOGY: linear
MOLECULE TYPE: other nucleic acid
ORIGINAL SOURCE: none
ORGANISM: none
STRAIN: none
FEATURE: primer for recognizing the nucleotides from numbers
1752 to 1767 on the neo gene; called "MI1511 neo"
SEQUENCE DESCRIPTION: SEQ ID NO: 3:

TCGTGGCTGG CCACGACGGG CGTTCC

SEQ ID NO: 4:
SEQUENCE LENGTH: 16
SEQUENCE TYPE: nucleic acid
STRANDEDNESS: single
TOPOLOGY: linear
MOLECULE TYPE: other nucleic acid
ORIGINAL SOURCE: none
ORGANISM: none
STRAIN: none
FEATURE: primer for recognizing the nucleotides from numbers
1846 to 1861 on the neo gene; called "MI1512 neo"
SEQUENCE DESCRIPTION: SEQ ID NO: 4:

GACAGGAGAT CCTGCC

## Claims

1. A method for preparing a sperm containing an exogenous DNA, wherein the exogenous DNA together with liposome for the purpose of transduction of DNA into mammalian cells is injected into a testis of a mature non-human vertebrate male and the sperm containing the exogenous DNA is produced in the test is of said male.

2. The method for preparing a sperm containing an exogenous DNA as claimed in claim 1, wherein the injection into a testis is repeated three times or more.

3. The method for preparing a sperm containing an exogenous DNA as claimed in claim 1 or 2, wherein said mature non-human vertebrate animal is a mouse.

4. A method for preparing a transgenic non-human vertebrate animal, wherein an exogenous DNA together with liposome for the purpose of transduction of DNA into mammalian cells is injected into a test is of a mature non-human vertebrate male and a sperm containing an exogenous DNA produced in the testis of said male is mated with an egg which has not been ferilized yet.

5. A method for preparing a transgenic non-human vertebrate animal, wherein an exogenous DNA together with liposome for the purpose of transduction of DNA into mammalian cells is injected into a test is of a mature non-human vertebrate and a sperm containing an exogenous DNA produced in the testis of said male is mated with an estrus female, making the female pregnant and delivered.

6. A method for preparing a transgenic non-human vertebrate animal, wherein an exogenous DNA together with liposome for the purpose of transduction of DNA into mammalian cells is injected into a testis of a mature non-human vertebrate and a sperm containing an exogenous DNA produced in the testis of said male is mated with a female,by artificial insemination or *in vitro* fertilization, making the female pregnant and delivered.

7. The method for preparing a transgenic non-human vertebrate animal as claimed in either one of the claims 4 to 6, wherein the injection into a testis is repeated three times or more.

8. The method for preparing a transgenic non-human vertebrate animal as claimed in either one of the claims 4 to 7, wherein said mature non-human vertebrate animal is a mouse.
